Europäisches Patentamt

European Patent Office

Office européen des brevets

(11) Publication number: **0 115 154**
A2

# (12) EUROPEAN PATENT APPLICATION

(21) Application number: **83307718.3**

(22) Date of filing: **19.12.83**

(51) Int. Cl.³: **C 12 P 19/06**

(30) Priority: **23.12.82 US 452671**

(43) Date of publication of application:
**08.08.84 Bulletin 84/32**

(84) Designated Contracting States:
**BE DE FR GB IT NL**

(71) Applicant: **PFIZER INC.**
**235 East 42nd Street**
**New York, N.Y. 10017(US)**

(72) Inventor: **Young, Thomas Benton**
**Box 151A RR2**
**Stonington Connecticut(US)**

(74) Representative: **Moore, James William**
**Pfizer Limited Ramsgate Road**
**Sandwich Kent CT13 9NJ(GB)**

(54) **Continuous production of xanthomonas biopolymer.**

(57) Continuous one- and two-stage processes for *Xanthomonas* fermentation are disclosed in which the chemically defined medium supplied to the first stage is growth-limiting with respect to nitrogen and metabolically balanced with respect to carbohydrate to ensure substantially complete consumption of both nitrogen and carbohydrate.

P.C. 6603 0115154

## CONTINUOUS PRODUCTION OF XANTHOMONAS BIOPOLYMER

There are extensive published reports relating to the production of hydrophilic colloids by the aerobic propagation of the bacteria of the genus Xanthomonas in aqueous nutrient media. Early work in this field was done at The Northern Regional Research Laboratory of The United States Department of Agriculture at Peoria, Illinois and is described in U.S. Pat. No. 3,000,790. Modified fermentation processes are described in U.S. Pat Nos. 3,020,206; 3,391,060; 3,427,226; 3,433,708; 3,271,267; 3,251,749; 3,281,329; 3,455,786; 3,565,763; 3,594,280; and 3,391,061.

The hydrophilic colloid (xanthan gum) produced by Xanthomonas campestris is a polysaccharide which has found wide food and industrial applications. Of special interest is the increasing focus on the use of xanthan gum in displacement of oil from partially depleted reservoirs.

The use of solutions of xanthan gum and its derivatives for oil recovery is described in U.S. Pat. Nos. 3,243,000; 3,198,268; 3,532,166; 3,305,016; 3,251,417; 3,391,060; 3,319,715; 3,373,810; 3,434,542 and 3,729,460. Techniques of permeability reduction are also used in enhanced oil recovery methods whereby Xanthan solutions are gelled by the addition of Cr(III) or other cations as described in U.S. Patent 4,048,079. It is suggested in U.S. Pat. No. 3,305,016 that aqueous solutions containing the heteropolysaccharide in sufficient quantity to increase the viscosity be employed as the thickening agent in preparing viscous waterflooding solutions. The polysaccharide may be prepared, separated, purified and then added. Alternatively, according to this reference, the entire culture, after adding a bactericide, may be added to the flood water.

U.S. Pat. No. 3,000,790 describes the culturing of a Xanthomonas bacterium in a medium containing glucose, an organic source of nitrogen, dipotassium phosphate and trace elements. The source of organic nitrogen usually employed is distillers' solubles, which contributes a substantial quantity of insolubles to the fermentation broth.

The processes described in U.S. Pat. Nos. 3,000,790, 3,391,060 and the other fermentation processes previously listed yield final fermentation broths that contain substantial amounts of insoluble matter, even when diluted with water for injection into oil-bearing subterranean formations. The particulate matter and in certain cases the Xanthomonas cells in such whole broth would soon plug the oil-bearing formation at the site of injection and thus foul the well and prevent any further oil recovery. Furthermore, the same problem would be encountered with reconstituted xanthan gum precipitated and separated from these fermentation broths. The plugging tendencies of these fermentation broths can be obviated by filtration, but such additional filtering steps are expensive and add significantly to the overall cost factors for enhanced oil recovery.

U.S. Pat. No. 3,853,771 approaches the plugging problem of whole fermentation broths by dissolving or dispersing cellular microorganisms by contacting said material with an aqueous solution containing surfactant effective for dispersing outer wall layers of microorganism cells, chelating agent for dispersing the inner wall layers of microorganism cells, and alkali metal hydroxide for enhancing said dispersing actions.

U.S. Pat. No. 4,010,071 describes a process for clarifying fermentation broths and other aqueous suspensions containing a dissolved xanthan gum and suspended solids by treatment with protease enzyme. However, this treatment does not overcome plugging problems due to insoluble inorganic or non-proteinaceous organic materials present in the fermentation medium or produced during the course of the fermentation.

The object of the process of U.S. Pat. No. 3,391,060 is to recover a polysaccharide product of substantial purity without the use of extensive separation procedures by the replacement of the organic source of nitrogen in the broth with an inorganic source (i.e., ammonium nitrate). However, the fermentation process described therein is not economical because of lengthy reaction times required and low yields of biopolymer obtained.

The method of improving the permeability of mobility control solutions by the addition of certain hydroxy substituted carboxylic acids is described in U.S. Pat. No. 2,867,279.

More recent process patents cover a fed-batch fermentation process (U.S. Pat. No. 4,282,321) and a semi-continuous fermentation process (U.S. Pat. No. 4,328,308 and 4,328,310) for xanthan production. A number of patents have dealt with production of xanthan by continuous fermentation. U.S. 3,228,262, describes a three stage continuous fermentation process. The first stage produces cell mass in a carbohydrate-free medium. The second and third stages are fed carbohydrate and produce xanthan biopolymer. Nitrogen is supplied in the first stage in complex organic form, which has the disadvantage of high cost and results in broths of poor filterability as detailed in U.S. Pat. No. 4,119,546. However, growth on carbohydrate free medium in the first stage is necessary in order to avoid "bacterial dissociation" of the Xanthomonas campestris strains employed.

U.S. Pat. No. 3,485,719 describes a single-stage nitrogen limited continuous fermentation which utilizes a similar complex form of organic nitrogen in feed medium. The patent employs a narrow range of dilution rate, carbohydrate source, and producing culture. Furthermore nitrogen limitation results in a loss of carbohydrate in effluent broth. This represents a significant economic penalty as there is no economic process for recovery of this major cost raw material. Another disadvantage of this process is that fermentation run time is limited to less than 8.7 turnovers due to the tendency of culture Xanthomonas campestris B1459 to degenerate with the feed medium employed.

U.K. Pat. No. 1,512,536 describes a one-stage continuous process employing a chemically defined medium and Xanthomonas growing under nitrogen, potassium, magnesium, phosphorus or sulfur limitation. The examples employ Xanthomonas juglandis.

U.K. Patent Application No. 2008,138A describes a single-stage continuous process employing chemically defined medium. Examples employ culture Xanthomonas campestris B1459 and do not address the problem of culture stability encountered in prior art. European Patent Application EPO 46,007 describes a single-stage continuous process employing a nitrogen-limited, chemically defined feed medium and a novel strain of Xanthomonas campestris. As demonstrated in Example 1 of said application, inferior specific polymer synthesis rates and culture degeneration result when culture B1459 is employed in this process. U.S. Patents 4,327,308 and 4,328,310 describe the use of non-degenerative strains of Xanthomonas campestris in a semi-continuous fermentation process. U.S. Patent No. 4,311,796 describes a method of improving specific productivity by growing Xanthomonas in a nitrogen-limited continuous fermentation in which cell concentration is 1.5-3.0 g/l and then increasing the nitrogen concentration to increase cell mass to not more than 5.0 gm/l. This process suffers substantial economic loss in the form of unconverted carbohydrate in effluent broth and low productivity during the first steady state period.

The present invention is directed to a prolonged steady-state process for producing heteropolysaccharide comprising the steps of

propagating a polysaccharide-producing <u>Xanthomonas</u> strain in a fermentation zone under aerobic conditions,

and continuously feeding a chemically defined aqueous nutrient medium to said zone at a rate substantially equal to the withdrawal rate of said fermentate,

said medium containing fermentable carbohydrate, an assimilable, inorganic nitrogen source and inorganic salts, and being growth limiting with respect to nitrogen and metabolically balanced with respect to carbohydrate whereby said carbohydrate and nitrogen source are substantially consumed in said process.

The process wherein feed and withdrawal rates are equivalent to a dilution rate of from about 0.03 to 0.15 $hr^{-1}$ and wherein the medium contains from about one to five per cent of said carbohydrate is preferred. The carbohydrate is preferably glucose, sucrose, maltose, fructose, lactose, starch or starch hydrolysate, especially glucose.

A medium nitrogen content of at least about 0.03 grams per liter and wherein said nitrogen source is ammonia or an ammonium salt such as ammonium sulfate, chloride or nitrate is preferred. Ammonia or ammonium sulfate are especially preferred.

A nitrogen:carbohydrate weight ratio in the medium of from about 0.015 to about 0.045 is preferred, as is the process wherein said Xanthomonas is Xanthomonas campestris. Moreover, there is a preferred optimal nitrogen:carbohydrate weight ratio for each species and/or strain.

The present process affords a fermentate product substantially free of insolubles of particle size above 3 microns and containing less than about 1 gram per liter of carbohydrate. With appropriate control of the relevant medium constituents, the fermentate product is likewise substantially free of soluble phosphorus, sulfur and trace metals and with metal cations of total weight less than 20 percent of the weight of heteropolysaccharide.

The present invention also includes a process wherein said fermentate withdrawn from the first fermentation zone above and nutrient medium consisting essentially of aqueous fermentable carbohydrate are each continuously introduced into a second aerated fermentation zone,

and fermentate is continuously withdrawn from said second zone at a rate substantially equal to the total of the feed rates to said second zone,

said feed and withdrawal rates being such that said fermentate withdrawn from said second zone has less than 3 grams per liter of soluble carbohydrate. The process wherein the fermentation volume in said second zone is from about one-half to three times the volume in the first zone and wherein the aqueous carbohydrate introduced to the second zone contains about 25 to 30% w/w carbohydrate is preferred.

Carbohydrate is preferably introduced to the second zone at the rate of from about 0.5 to 1.5 grams per liter per hour and the carbohydrate is preferably glucose, sucrose, maltose, fructose, lactose, starch or starch hydrolysate, especially glucose.

The fermentate product of this two-step process substantially free of insolubles of particle size above 3 microns and containing less than about three grams per liter of carbohydrate is yet another part of this invention. The product substantially free of insoluble phosphorus, sulfur and trace metals and with metal cations of total weight of less than 20 percent of the heteropolysaccharide present is preferred. The fermentate product of either the one-stage or two-stage processes above is eminently suitable for crude oil recovery from oil-bearing subterranean formations.

As explained in U.K. Patent No. 1,512,536 the limiting nutrient in a feed medium is usually supplied in a quantity equivalent to the fraction of that nutrient contained in the cell mass produced in effluent broth, while other nutrients are added in considerably greater quantity than that required for growth. In the past continuous processes employing Xanthomonas have been developed in which growth was limited by carbon, nitrogen, sulfur, potassium and other nutrients. In the process of this invention I employ a feed medium in which growth is limited by nitrogen, but unlike prior art I add other nutrients, particularly carbohydrate, at the minimal level required to effect the desired cell growth and polymer synthesis in a one or two stage continuous process. Of these other nutrients the carbon source is, of course, of major economic importance and an optimum C/N ratio can be established for each <u>Xanthomonas</u> strain to saturate the polymer synthesizing ability of the organism growing in a continuous process while simultaneously eliminating residual carbohydrate in effluent and achieving superior carbohydrate conversion efficiency.

By employing a metabolically balanced feed medium a number of unexpected benefits accrue. Firstly, with strains of <u>Xanthomonas</u> <u>campestris</u> hitherto considered "unstable", steady states of high productivity and superior yield can be maintained for at least 17 turnovers. Secondly, employment of balanced feed medium with respect to carbohydrate, nitrogen and salts results in fermentation broths of considerably lower viscosities than those containing excessive residual nutrients and salts. Lower broth viscosity in turn allows improved mass transfer rates and results in higher productivity without additional energy input costs. Finally, continuous metabolically balanced feed medium produces whole broths with superior filterability properties when employed in mobility control solutions. Representative species of the genus which may be utilized in accordance with the invention include <u>Xanthomonas</u> <u>begoniae</u>, <u>Xanthomonas</u> <u>campestris</u>, <u>Xanthomonas</u> <u>carotae</u>, <u>Xanthomonas</u> <u>hederae</u>, <u>Xanthomonas</u> <u>incanae</u>, <u>Xanthomonas</u> <u>malvacearum</u>, <u>Xanthomonas</u> <u>papavericola</u>, <u>Xanthomonas</u> <u>phaseoli</u>, <u>Xanthomonas</u> <u>pisi</u>, <u>Xanthomonas</u> <u>translucens</u>, <u>Xanthomonas</u> <u>vasculorum</u> and <u>Xanthomonas</u> <u>vesicatoria</u>.

Preferred conditions for fermentation in both the first and second stages are as follows:

| | |
|---|---|
| temperature | 28-32°C |
| pH | 6.0-7.0 |
| aeration rate | .5 - 1.5 v/v/min. |
| agitation | 600-1000 rpm (lab scale) |

Those skilled in the art will realize that conditions, especially agitation, must be adjusted for the scale of the fermentation being conducted.

The examples to follow serve to illustrate but do not limit the scope of this invention.

## Comparative Example

A two liter Fernbach flask containing 500 ml of sterile medium of Table 1 was inoculated with Xanthomonas campestris NRRL B1459 and grown at 30°C and 250 rpm for 24 hours. A 5 liter fermentor containing 2 liters of the medium described in Table 2 was inoculated with 400 ml of culture taken from the Fernbach and allowed to grow in batch mode for 36 hours under the conditions of Table 3. At that time a feed was begun at 100 gm/hr with feed medium of the same composition as batched and working volume was reduced to 2 liters. After 80 hours of operation a steady state was achieved as summarized in Table 4. After 6.5 turnovers or at 160 hours the culture lost ability to grow and make polymer. At 260 hours of operation cell and polymer concentrations in effluent were less than 10% of steady state values. Noting from Table 4 that glucose level at steady state was greater than 1.5 gm/l, the culture degeneration observed is consistent with prior art in which nitrogen limited growth and excessive residual glucose was employed with B1459 culture (Rogovin, Biotechn. & Bioeng.. 14: p. 23, 1972, and European Patent Application EPO 46,007).

## Table 1
### Medium for Fernbach Flask Comparative Example

|  | gm/l |
|---|---|
| glucose (sterilize separately) | 30 |
| ammonium nitrate | 1.0 |
| potasssium phosphate monobasic | 0.69 |
| potassium phosphate dibasic | 4.10 |
| magnesium sulfate .7H$_2$O | 0.10 |
| manganese sulfate .H$_2$O | 0.03 |
| ferrous sulfate .7H$_2$O | 0.01 |
| calcium chloride .2H$_2$O | 0.2 |
| citric acid | 1.0 |

sterilize 30 minutes at 121°C. Combine glucose and salts aseptically after cooling.

## Table 2
### Feed Medium for Comparative Example

|  | gm/l | Table 2a gm/l |
|---|---|---|
| glucose (sterilize separately) | 20 | 18 |
| ammonium sulfate | 2.0 | 1.8* no pH adj. |
| potassium phosphate monobasic | 0.4 | 0.4 |
| citric acid | 1.0 | 1.0 |
| magnesium sulfate .7H$_2$O | 0.2 | 0.2 |
| manganese sulfate .H$_2$O | 0.06 | 0.06 |
| ferrous sulfate .7H$_2$O | 0.02 | 0.02 |
| calcium chloride .2H$_2$O | 0.2 | 0.2 |

*ammonia

Adjust pH of salts to 6.6 with sodium hydroxide before sterilization. Sterilize 1 hour at 121°C. Combine glucose and salts aseptically after cooling.

In Examples 1-5 fermentor operating conditions are given in Table 3:

### Table 3
### Operating Conditions for First Stage & Second Stage
### Continuous Xanthan Fermentations

| | |
|---|---|
| Temperature | 30°C |
| pH | 6.4 +0.1 automatic addition sodium hydroxide |
| Aeration | 1.0 vvm |
| Agitation | 800-1000 rpms |

### Example 1

A 5 liter fermentor with 2 liter working volume was inoculated with a culture of NRRL B1459 as in the Comparative Example. Batch makeup and feed medium were identical with Table 2 except that glucose level was reduced to 18 gm/l. After 48 hours of batch growth glucose concentration was below 1 gm/l and feed was begun at 85 gm/hr and gradually increased to 100 gm/hr by 113 hours. A steady state was achieved as summarized in Table 4 and was maintained until 207 hours at which time feed medium concentration was increased 25%. A second steady state was reached and maintained until 337 hours as summarized in Table 4. Note that in this example residual glucose during continuous operation was less than 1.0 gm/l. A total of 14.5 turnovers was observed.

## Example 2

A 5 liter fermentor was operated identically with Example 1 for 17.4 turnovers, the total operation time being 330 hours. Steady state performance is shown in Table 4.

## Example 3

A 5 liter second stage fermentor with working volume of 2 liters was batched with medium, inoculated, and operated in batch mode for 40 hours identically and simultaneously with the first stage fermentor of Example 2. At that time effluent broth was pumped from the fermentor of Example 2 into the second stage fermentor, working volume was adjusted to two liters, and effluent from the second stage was removed at the same rate as received from the first stage to maintain the 2 liter volume. A 25% wt/wt glucose feed was begun at 5.5 gm/hr. At 60 hours a steady state was obtained in which broth effluent was removed at a rate equal to the sum of first stage effluent removal rate and second stage glucose feed rate as summarized in Table 4 and was maintained for 17.4 turnovers, the total operation time being 330 hours. Note residual glucose in the second stage was 1-2.0 gm/l. As cell growth was minimal in Stage 2 no instability was observed.

## Example 4

A two liter Fernbach flask containing 500 ml of sterile medium of the following composition:

|  | gm/l |
|---|---|
| Soytone | 15 |
| Bactopeptone | 15 |
| Glucose | 10 |

Sterilize 30 minutes at 121°C.

was inoculated with <u>Xanthomonas</u> <u>campestris</u> NRRL B1459 and grown at 30°C and 250 rpm for 48 hours. Two liters of medium as described in Example 1 of U.K. Patent 1,512,536 was then batched in a 5 liter fermentor, sterilized and inoculated with 400 ml of broth taken from the Fernbach flask. The fermentor was run according to the conditions of Example 1 of U.K. patent 1,512,536 in batch growth for 33 hours at which time feed of the same composition as batched was begun at 85 gm/hr. Cell growth as measured by optical density reached 5 gm/l in batch growth and decreased to 1.15 gm/l at 120 hours of operation. Polymer concentration peaked at 58 hours after which it decreased to below 0.5% at 120 hours. Glucose concentration at the end of batch growth was 1.0 gm/l and increased to 15 gm/l at 120 hours. In summary batch medium enriched with inoculum carryover of nutrients sustained cell growth but feed medium did not. At 120 hours 2 liters of medium given in Table 2a was rapidly added to the fermentor while simultaneously removing an equal amount. Batch growth was allowed until 160 hours at which time continuous feeding of medium from Table 2a was resumed at 80 gm/hr and increased to 122 gm/hr.

A steady state was achieved as summarized in Table 4 and maintained from 160 to 380 hours. At 384 hours nitrogen and sulfur concentration in feed was increased to achieve a nitrogen to glucose ratio of 0.035 approximating the ratio employed in Example 2 of U.K. Patent Application 2008,138A. As cell growth was nitrogen-limited at the earlier nitrogen to glucose ratio of 0.0226, the cell concentration as measured by optical density OD increased from 7.5 to 9.0.

As cell concentration increased polymer concentration decreased from 7.1 to 5.7 gm/l indicating increased carbon for cell growth had come at the expense of polymer production representing a 23% loss
in carbohydrate conversion efficiency. At 456 hours
of operation feed medium of composition given in Table
2a, but at one and one half times higher concentration
was employed. A steady state was achieved within 20
hours and maintained for 220 hours with a performance
given in Table 4. A total of 27 turnovers was observed
employing metabolically balanced feed medium. This
example demonstrates the unsuitability of the feed
medium of U.K. 1,512,536 for maintaining growth in
continuous culture of Xanthomonas campestris NRRL
B1459, the fact that the "carbon limited" conditions
described in U.K. Application 2,008,138A are in fact
nitrogen limited and that Example 2 of that application is inefficient with respect to xanthan yield
from carbohydrate. By contrast, conditions described
in the present invention give good xanthan yield from
carbohydrate and are nitrogen limited with respect to
growth. In addition the use of ammonia as illustrated
in this example, Table 2a and Table 4 results in
reduction of salinity due to sodium and sulfate
residues. Sodium levels are reduced because the
higher pH of the feed medium ( 8.8) reduces the amount
of sodium hydroxide required for pH control. Sulfate
levels are reduced due to the elimination of the
sulfate anion from the nitrogen source $[(NH_4)_2SO_4]$.
This reduced salinity results in a reduction of broth
viscosity which is desirable from the mass transfer
and mixing standpoint.

## Table 4: Steady State Performance Parameters for Continuous Fermentations

| | Comparative Example | Example 1 SS1 | Example 1 SS2 | Example 2 *SS1 | Example 3 SS1 | Example 4 SS1 | Example 4 SS2 |
|---|---|---|---|---|---|---|---|
| Cell Concentration, gm/l | 2.5 | 2.64 | 3.79 | 2.5 | 3.0 | 2.5 | 3.46 |
| Glucose Concentration, gm/l | 1.5 | <1.0 | <1.0 | <1.0 | 1-2.0 | <1.5 | <1.0 |
| Polymer Concentration, gm/l | 7.7 | 7.8 | 8.5 | 7.0 | 12.5 | 7.0 | 9.2 |
| Specific Rate, gm/gm-hr | 0.15 | 0.16 | 0.12 | 0.15 | 0.10 | 0.16 | 0.15 |
| Volumetric Rate, gm/lhr | 0.38 | 0.41 | 0.43 | 0.38 | 0.29 | 0.41 | 0.51 |
| Conversion Efficiency, gm/gm | 0.38 | 0.43 | 0.37 | 0.39 | 0.38 | 0.39 | 0.34 |
| Viscosity, cps | 3600 | 3500 | 4800 | 3300 | 6500 | 2500 | 4200 |
| Dilution Rate, $hr^{-1}$ | .051 | .053 | .051 | .054 | .054 | .058 | .055 |

*SS = Steady state

## Example 5

Broths taken from fermentor effluents in Examples 1-4 when operating at steady state with balanced feed medium and tested for filterability according to the test procedure given in U.S. Patent 4,119,546 gave the following results:

### Filterability of Xanthan Broths through 1.2  Millipore Filters in 500 ppm TDS* Solution

| | |
|---|---|
| Example 1 | 1.18 |
| Example 2 | 2.52 |
| Example 3 | 1.10 |
| Example 4 | 1.60 |

*total dissolved salts.

## Example 6

Broth from Example 3 was taken from effluent at steady state, diluted and ultrafiltered through a 50,000 molecular weight cutoff hollow fiber membrane to remove water and salts from cells and polymer. Analysis of the permeate by atomic absorption and colorimetric techniques and correction for dilution gave the results shown below:

### Analysis of Residual Salts in Broth Example 3 (SS1)

| | ppm |
|---|---|
| Sodium | 840 |
| Potassium | 123 |
| Phosphorus | 42 |
| Magnesium | 4.8 |
| Manganese | 8.3 |
| Iron | 4.0 |
| Calcium | 4.2 |

0115154

P.C. 6603

## CLAIMS

1.   A prolonged steady-state process for producing heteropolysaccharide comprising the steps of
propagating a polysaccharide-producing Xanthomonas strain in a fermentation zone under aerobic conditions,
and continuously feeding a chemically defined aqueous nutrient medium to said zone at a rate substantially equal to the withdrawal rate of said fermentate,
said medium containing fermentable carbohydrate, an assimilable, inorganic nitrogen source and inorganic salts, and being growth limiting with respect to nitrogen and metabolically balanced with respect to carbohydrate whereby said carbohydrate and nitrogen source are substantially consumed in said process.

2.   The process of claim 1 wherein said feed and withdrawal rates are equivalent to a dilution rate of from about 0.03 to 0.15 $hr^{-1}$.

3.   The process of Claim 1 wherein said medium contains from about one to five per cent of said carbohydrate selected from glucose, sucrose, maltose, fructose, lactose, starch and starch hydrolysate.

4.   The process of Claim 3 wherein said carbohydrate is glucose.

5.   The process of Claim 1 wherein said medium contains at least about 0.03 grams nitrogen per liter, said nitrogen source being ammonia or an ammonium salt selected from ammonium sulfate, nitrate or chloride.

6. The process of Claim 1 wherein the nitrogen: carbohydrate weight ratio of said medium is from about 0.015 to about 0.045.

7. The process of Claim 1 wherein said <u>Xanthomonas</u> is <u>Xanthomonas campestris</u> strain B1459A.

8. The fermentate product of the process of Claim 1 substantially free of insolubles of particle size above 3 microns and containing less than about 1 gram per liter of carbohydrate.

9. The product of Claim 8, substantially free of soluble phosphorus, sulfur and trace metals and with metal cations of total weight less than 20 percent of the weight of heteropolysaccharide present.

10. The process of Claim 1 wherein (a) said fermentate withdrawn from the first fermentation zone and (b) nutrient medium consisting essentially of aqueous fermentable carbohydrate are each continuously introduced into a second aerated fermentation zone, wherein the fermentation volume in said second zone is from about one-half to three times the volume in said first zone,

and fermentate is continuously withdrawn from said second zone at a rate substantially equal to the total of the feed rates to said second zone,

said feed and withdrawal rates being such that said fermentate withdrawn from said second zone has less than 3 gram per liter of soluble carbohydrate.

11.    The process of Claim 10 wherein said carbohydrate is introduced to said second zone at the rate of from about 0.5 to 1.5 grams per liter per hour and said carbohydrate is glucose.

12.    The fermentate product of the process of Claim 10 substantially free of insolubles of particle size above 3 microns, containing less than about three grams per liter of carbohydrate substantially free of soluble phosphorus, sulfur and trace metals and with metal cations of total weight less than 20 percent of the weight of the heteropolysaccharide present.